# EUROPEAN PATENT APPLICATION

(11) **EP 4 706 742 A1**
(43) Date of publication of application: **11.03.2026**
(21) Application number: 24198473.1
(22) Date of filing: 04.09.2024
(51) Int. Cl.: A61M 60/148, A61M 60/837, A61M 60/268, A61M 60/454

(54) **IMPLANTABLE PUMP**

(71) Applicant: Ecole Polytechnique Fédérale de Lausanne (EPFL), 1015 Lausanne (CH)
(72) Inventor: Benouhiba, Amine, 2000 Neuchâtel (CH); Civet, Yoan, 74500 Publier (FR); Perriard, Yves, 2000 Neuchâtel (CH); Walter, Armando, 2000 Neuchâtel (CH)
(74) Representative: P&TS SA (AG, Ltd.)

(57) **Abstract**

The present invention relates to a pump (1) implantable in a human or animal body and comprising :
an airtight casing (10),
a dielectric elastomer tube (20) extending within said casing (10), arranged so that a volume (22) within said dielectric elastomer tube (20) is modified when a voltage is applied to said dielectric elastomer tube;
a negative pressure chamber (N) between said dielectric elastomer tube (20) and said casing (10).

The casing comprises two lateral edges (100a, 100b). The dielectric elastomer tube (A) is longitudinally pre-strained between those lateral edges.

## Description

### Technical domain

The present invention relates to an implantable pump comprising a dielectric elastomer tube as an actuator. Such a pump is adapted to pump a fluid flow in a conduit, such as the blood flow in an artery, in particular for cardiac assistance.

### Related art

US8016739 describes an aortic compression device for increasing coronary blood flow. The device alternately compresses and decompresses the aorta of the heart to be assisted. It comprises a pump adapted to pump fluid from a reservoir in the patient's chest to the compression device. It is difficult to implant a pump, a compression device and a fluid reservoir in the body of a patient.

A pump requiring fewer components is described in US7064472. This document proposes an electroactive polymer film whose dimensions are controlled by applying a voltage to electrodes on either side of the film. The deformation is used to pump a fluid.

WO2021220087A1 describes a tubular stretchable device, the viscoelasticity of which can be modified when a given voltage is applied, resulting in a modification of the retractable force. Such a tubular device, when used as an implant around or in place of a portion of an artery, expands under the pressure of the blood flow and retracts naturally when the blood flow decreases. By modifying its viscoelastic properties by applying a specific voltage, its expansion can be adjusted, in particular to facilitate its expansion under blood pressure. When the applied voltage is removed, the natural retraction force is restored. The expansion of the tubular device is controlled by the pressure of the blood flow inside the artery against the pressure outside the artery. The expansion of the device is facilitated during the diastolic phase of the heart, allowing an increase in coronary blood flow and supporting the heart. The device as such is not a pump.

Pumps based on electroactive polymers are compact and reliable and therefore well suited to controlling fluid displacement within a living body. US7128707 discloses an artificial sphincter based on such an electroactive polymer. US7371223 discloses an electroactive polymer actuated heart-lung bypass pump.

The energy that can be delivered by dielectric elastomeric tubing depends on, among other things, its thickness and the voltage applied. However, it has been found that the head pressure and fluid flow required for applications such as cardiac assist require either a very thick tube, a very high voltage, or both. It would be desirable to reduce the thickness and voltage required in a pump implanted in the body.

US7411331 discloses another actuator comprising an elastomeric film coated with electrodes on both surfaces. The system is actuated by applying a voltage to the electrodes which causes the film to compress in thickness and expand in surface area. To avoid the high voltages that would be required to achieve high pressure, the document suggests pre-straining the dielectric material.

An actuator that provides a more uniform force over the displacement range is described in US7411331, which suggests compensating for the positive stiffness of the electroactive polymer film with a spring that has a constant and negative stiffness over a certain range. Coupling an electroactive polymer film with a pre-strained spring having a negative stiffness increases the amount of displacement that can be achieved for a given voltage or energy.

WO2019/102417 describes another implantable ventricular assist device comprising an electroactive polymer tube and a spring for pre-straining the polymer.

Implantation of an actuator comprising an electroactive polymer tube and a metallic spring in a human or animal body presents a number of difficulties. It would be desirable to remove the spring or at least to require a smaller or weaker spring.

The document entitled "dielectric elastomers pump for artificial organisms", Proc. SPIE 7976, and Devices (EAPAD) 2011, by A. Bowers et al. discloses a pump comprising an electroactive polymer tube which is held in place by a vacuum outside the tube. The device is intended for use on board a microbial fuel cell powered robot. Despite being relatively bulky, its flow rate is limited to a few µl/s, making it unsuitable for applications such as cardiac support. The flow of the pumped fluid is regulated by valves, so it cannot be applied to an artery where the blood already has a natural flow cycle due to the heart contractions.

### Brief disclosure of the invention

One object of the present invention is to provide a pump implantable in a human or animal body which overcomes or mitigates the disadvantages of the prior art.

One object of the present invention is to provide a design for a pump that allows, with suitable dimensioning, to achieve the head pressure and flow rate required for an aortic assistance pump, in a volume adapted for implantation in a human body. Such an application typically requires a flow rate of more than 2000mL/min, preferably more than 4000mL/min, and a head pressure of more than 30mmHg, preferably more than 40mmHg. To be implantable, the length of the pump should be less than 80mm, preferably less than 60mm, and its diameter less than 50mm, preferably less than 40mm.

A further object of the present invention is to provide a method of assembling a cardiac assist device according to the present disclosure on an artery.

According to the invention, these objectives are achieved by the subject matter of the independent claims and are described in detail in the dependent claims.

In particular, these aims are achieved by a pump implantable in a human or animal body comprising :
an airtight casing;
a dielectric elastomer tube extending within said casing, arranged so that a volume within said dielectric elastomer tube is modified when a voltage is applied to said dielectric elastomer tube;
a negative pressure chamber between said dielectric elastomer tube and said casing;
wherein said casing comprises two lateral edges and in that said dielectric elastomer tube is longitudinally pre-strained between said lateral edges.

The negative pressure chamber is thus used to pre-strain the dielectric elastomer tube in a substantially radial direction. The lateral edges are used to pre-strain the dielectric elastomer tube in a predominantly axial direction. Both pre-strains increase the head pressure and flow rate that can be achieved for a given voltage or energy. Separating the radial and axial pre-strains allows better control of the tube strains and of its deformations.

With respect to what is known in the art, the invention provides the advantage of a pump that can extract more energy from the dielectric elastomer tube than prior art pumps, by pre-stretching the tube in both longitudinal and radial directions, thus converting more energy into fluid energy. This results in higher flow rates and/or higher head pressure compared to prior art pumps employing a dielectric elastomer tube of similar size.

The vacuum within the negative pressure chamber serves as a pneumatic biasing system (pneumatic spring), allowing for the selection of the dielectric elastomer walls thickness to be independent of external factors, thus creating a complete, self-contained actuator that is free from external influences.

The invention also provides the advantage of providing a pump that requires less volume than existing body implanted pumps providing a similar flow rate and head pressure.

### Brief description of the drawings

Exemplary embodiments of the invention are disclosed in the description and illustrated by the following drawings:
- Figure 1: schematic perspective representation of an implantable pump according to an example of the present invention,
- Figure 2: schematic perspective and sectional view of an implantable pump according to an example of the present invention,
- Figure 3: schematic perspective and sectional view of the casing of an implantable pump according to an example of the present invention,

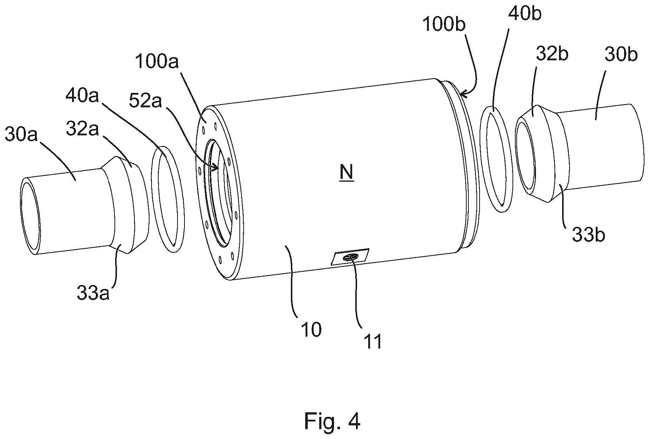

- Figure 4: schematic perspective representation of some components of an implantable pump according to an example of the present invention,

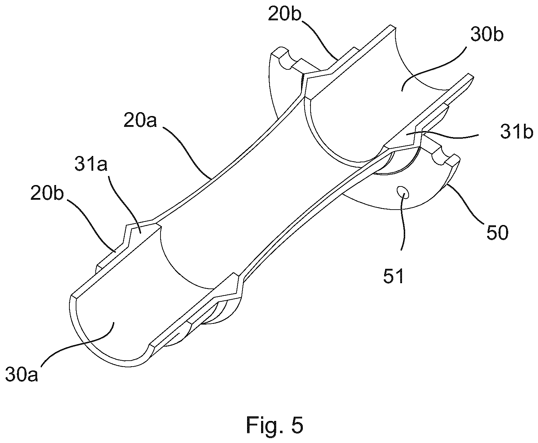

- Figure 5: schematic perspective and sectional view of an implantable pump according to an example of the present disclosure without a casing,

### Examples of embodiments of the present invention

The device 1 according to the present invention is a body implantable pump, for example a ventricular assistance device.

With regard to the figures, the present device comprises an airtight casing 10 in which a dielectric elastomer tube 20 is arranged.

The casing may have any suitable shape. As an example, the casing 10 shown herein is cylindrical in shape. This is however not intended to limit the scope of the present disclosure. For example, casings having a square, ellipsoidal, oval, rectangular or polygonal cross section could also be considered.

The casing 10 is preferably rigid or at least semi-rigid so as to resist to the surrounding pressure and pressure variations, as well as internal negative pressures. It can be made for example with a body compatible rigid polymer, a metal, or an alloy. The pump 1 being expected to be implanted in a patient's body, it should also resist to the oxidation. Material of surgical quality should therefore be preferred. For example the casing 10 can be made of titanium or stainless steel of surgical grade or hard polymer of surgical grade.

The casing provides a protection for the dielectric elastomer tube 20 against the surrounding environment. The dielectric elastomer tube 20 is thus not in direct contact with the surrounding environment. The surrounding environment includes all the organs, fluids, and biological elements such as muscles, vessels, nerves nearby the artery on which the present device 1 is mounted. The casing 10 thus provides a volume 12 forming a chamber N in which the dielectric elastomer tube 20 can extend. Beside the mechanical protection, the casing 10 also avoids biological cells to adhere to the surface of the dielectric elastomer tube 20, which may alter its performances over time.

According to one embodiment, the dielectric elastomeric tube 20 may include some or all of the features of the dielectric elastomeric tube disclosed in patent application WO2021220087A1, the entire content of which is incorporated by reference.

The dielectric elastomer tube 20 may comprise one or preferably several layers of an electroactive polymer or a mixture of electroactive polymers such as silicone or other known electroactive polymers. Electrodes are disposed on each side of each layer of polymer. A voltage can be applied between the electrodes to modify the viscoelastic properties and/or the dimensions of the polymer layer. This voltage application is used to expand the internal volume 22 or the tube or to compress it to expel the fluid within the volume 22.

The thickness of the dielectric elastomer tube is preferably higher than 1mm, for example between 2.4 and 4mm, more preferably between 3 and 4mm. This range provides a good trade-off between small dimensions for human body implantation and required flow rate/pressure.

The dielectric elastomer tube 20 comprises electrical connection means, such as electrical wires, arranged at one or both of its ends. A portion of the electrodes is preferably arranged outside the casing 10, once the dielectric elastomer tube 20 is properly mounted inside the casing. The connection means allow the extensible device 20 to be powered by an energy source such as a battery.

A voltage may be applied to the electrodes at predetermined frequencies. It can be synchronised with the heart pulsations.

The dielectric elastomer tube 20 can be used, for example, as an aortic counterpulsation device. It can store elastic energy during systole and release it during diastole.

The casing 10 can further comprise two lateral edges (or flanges) 100a, 100b. As will be described, the dielectric elastomeric tube 20 is held and longitudinally restrained between these lateral edges.

The lateral edges 100 are preferably integral with the remainder of the casing 10, so that the casing 10 as a whole forms a hollow tube, both ends of which are partially closed by the lateral edges 100. According to a preferred arrangement, the lateral edges 100 are arranged orthogonally to the central portion of the casing 10 surrounding the artery. In other words, the lateral edges 100 are also orthogonal to the blood flow F when the device is placed on an artery. However, this does not exclude embodiments in which the lateral edges 100 are arranged at an angle other than 90° with respect to the central portion of the casing 10, depending on the requirements.

Each of the lateral edges 100a, 100b comprises a casing opening 52a, 52b, preferably located at a central position of the corresponding lateral edge.

A mouthpiece (or tubular connector) 30a, 30b is inserted in each opening 52a, 52b.

These mouthpieces 30a, 30b are used to connect the two ends of a cut artery in order to introduce the pump 1 into the blood flow F. The mouthpieces 30a, 30b preferably have a circular section with a suitable diameter so as to be embedded in the artery. This does not exclude that they are slightly conical in order to facilitate insertion into the artery. It also does not preclude other shapes or surface treatments or means for proper maintenance of the artery associated with the mouthpieces 30a, 30b.

The mouthpieces 30a, 30b may be made of or comprise a hard material such as ceramic, polymer, metal and/or a soft material such as silicon or an equivalent soft polymer. In one embodiment, the proximal portion of the mouthpieces at the side edges 100a, 100b is made of a hard material and the distal end is made of a softer material.

The mouthpieces 30a, 30b are preferably removably attached to the casing 10. This makes it possible to adapt mouthpieces of different diameters to a casing. This also makes it possible to connect each of the mouthpieces 30a, 30b at the ends of a cut artery and then in a subsequent step to connect the mouthpieces 30a, 30b to the casing 10.

Even if the pump 1 is assembled outside the patient's body before being implanted, the assembly of removable parts makes it possible to adjust certain parameters during assembly. In particular, the pre-strain of the tube 20 can be adjusted at the time of implantation of the pump 1.

The dielectric elastomer tube 20 is intended to replace a portion of an artery, such as the aorta, or of any other body conduit through which a fluid must circulate. In this way, the blood flow F of an artery can enter the device 1 through one of the mouthpieces and leave the pump 1 through the opposite mouthpiece. The diameter of the mouthpiece is adapted to the size of the artery on which the pump 1 is to be placed. The mouthpieces are interchangeable and can be adapted to different diameters of arteries or other body tubes. Dimensions such as the length and diameter of the mouthpiece can therefore be adapted, as can the material from which they are made. A pump can be manufactured and sold with a set of different mouthpieces of different dimensions and/or materials.

The outer surface of each mouthpiece 30a, 30b comprises a circumferential projection, or rib, 31a, 31b forming two opposed surfaces 32a, 32b.

While these two opposing surfaces 32a, 32b may be parallel to each other, they are preferably angled so as to form a V-shape. The two opposite surfaces 32a, 32b can form an angle between 10° and 160°, preferably between 20° and 140°, or between 40° and 120°, such as about 70°, 80° or 90°. Whilst the two opposing surfaces 32a, 32b may form a straight edge, it is preferable for the angle formed to be rounded so as to provide a smooth surface. This will prevent the stretchable device 20 from being damaged when placed over the projection 31a, 31b, as will be more fully described below. The opposed surfaces 32a, 32b need not be symmetrical. One of the opposing surfaces 32a, 32b may be more inclined than the other.

Instead of a circumferential projection 31a, 31b, on could use a plug or a pin or any type of projection facing outwards from the corresponding mouthpieces 30a, 30b to connect it to the casing.

The projections 31a, 31b are preferably integral with the corresponding mouthpieces 30a, 30b. They may also be removably disposed on the outer periphery of the mouthpieces.

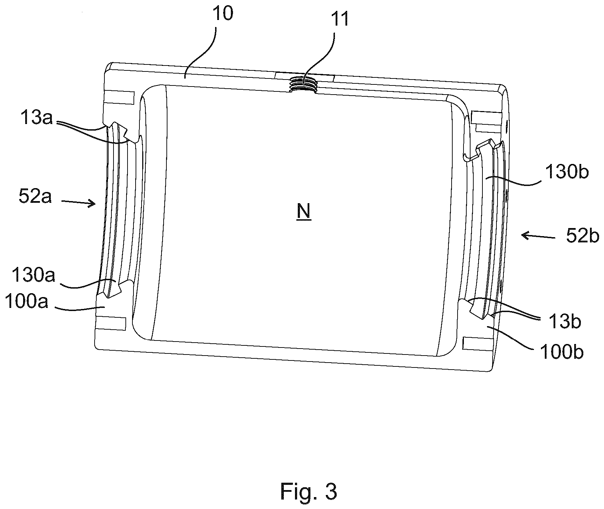

As can best be seen in Figure 3, each of the openings 52a, 52b through the lateral edges 100a, 100b of the casing 10 defines a first surface 13a, 13b respectively. Such a surface 13 is oriented so as to correspond to one of the opposite surfaces 32a, 32b of the mouthpieces 30a, 30b. When the opposed surfaces 32a, 32b are angled with respect to each other, the surface 13 is bevelled so as to correspond to the corresponding surface 32a, 32b of the mouthpiece 31a, 31b. The bevel of the first surfaces 13a, 13b is preferably directed outwards from the corresponding lateral edge 100.

Each end 20b of the dielectric elastomer tube 20 is placed around one of the mouthpieces 30a, 30b and pressed with its surface 32a, 32b against a first surface 13a, 13b of the opening 52a, 52b. The dielectric elastomer tube 20 is held against the mouthpieces at each of its ends.

The compression of the dielectric elastomer tube 20 between the surfaces 13 and 32 provides a watertight seal.

One or both of the surfaces 13, 32 may be provided with a recess for receiving a seal such as an O-ring 40a, 40b. Figure 3 shows a recess 130 within the first surfaces 13a and 13b of the side edge 100a and 100b respectively. Alternatively or in addition, a similar recess may be formed in the surface 53 of the locking ring 50. Alternatively or additionally, similar recesses can be made in one or both of the opposing surfaces 32, 33 of the circumferential projections 31a, 31b of the mouthpieces.

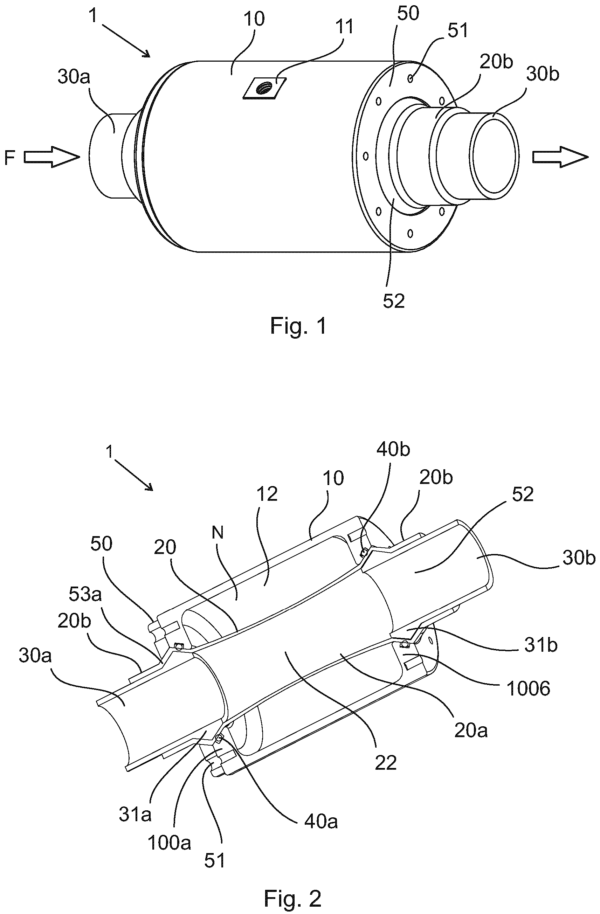

According to one embodiment, at least one mouthpiece 30a, 30b is pressed against the dielectric elastomer tube 20 and against the first surface 13a, 13b by a locking ring 50 shown in Figure 2.

The section of the locking ring 50 preferably corresponds to the section of the lateral edges 100 of the casing 10.

The locking ring 50 is provided with a central hole adapted to face the corresponding aperture 52a or 52b. The central hole defines a second surface 53a, 53b corresponding to the second of the opposed surfaces 33a, 33b of the mouthpieces 30a, 30b. When the opposed surfaces are V-shaped, the second surface 53a, 53b of the locking ring 50 is bevelled at a suitable angle to face the corresponding surface 33a, 33b. In particular, the bevel of the surface 53 is directed inwards so that the locking ring can be moved towards the side edge 100 until the second surface 53a, 53b comes into contact with the dielectric elastomer tube 20 opposite the corresponding opposite surface 33a, 33b of the mouthpiece.

The pump 1 further comprises clamping means 51 adapted to clamp the locking ring 50 against the corresponding lateral edge 100 of the casing 10. Such tightening clamps the two opposite surfaces 32a, 33a of the mouthpieces 30a, 30b so as to mechanically connect the mouthpieces to the casing 10 and to compress the dielectric elastomer tube 20 between the mouthpiece on the inside and the surfaces 13, 53 on the outside.

The clamping means 51 may comprise, for example, threaded holes located on the side edges 100 and through holes located in the locking rings and facing the threaded holes of the side edges 100 so that screws can be inserted over the locking ring 50 and tightened against the corresponding side edge 100. Such an arrangement is not intended to be limiting and other suitable clamping means could be considered, such as clips or equivalent means.

According to one embodiment, the locking rings 50 are formed from a single piece. In this case, the locking rings 50 can be engaged around the corresponding mouthpieces and can slide axially until they are in contact with the lateral edges 100.

According to another embodiment, the locking rings 50 are made of two or more distinct pieces. For example, two half rings can be envisaged. In this way, the casing 10 can be placed between the mouthpieces 30a, 30b so that the first surface 13 is in contact with the corresponding surface 310, 311 of the mouthpiece, and then the half rings can be arranged from the sides of the mouthpieces and tightly against the corresponding lateral edge 100.

As for the casing 10, each locking ring 50 should meet the requirements of the surgery. It may be made of a hard polymer, a metal or an alloy suitable for such an implantable device.

A locking ring may be provided on one side of the casing or on each side.

The dielectric elastomer tube 20 is held between the two mouthpieces 30a, 30b with a suitable longitudinal pre-strain. The extremities of the stretchable device 20 are thus properly maintained on the corresponding mouthpieces 30a, 30b so as to avoid any sliding or disassembly.

The longitudinal pre-strain increases the volume of fluid that can be pumped per cycle for a given tension and thickness of the dielectric elastomer. It also prevents the risk of the tube bending when a voltage is applied.

This longitudinal pre-strain is determined by the elongation of the tube 20 when it is mounted and prevented from sliding, for example by clamping the locking ring 50.

The longitudinal pre-strain can be continuously adjusted by pulling on the dielectric elastomer tube during assembly. The strain can be adjusted individually for each tube, compensating for manufacturing tolerances. It can be measured using a suitable force sensor. Alternatively or additionally, reference marks may be provided on the dielectric elastomer tube 20 so that it is properly aligned with corresponding marks or portions of the casing or mouthpiece. Alternatively or additionally, one or both of the mouthpieces 30a, 30b may be provided with elongation adjustment means for adjusting the elongation of the stretchable device 20 before or after it is clamped to the casing 10. Such means may include, for example, a ring to which the stretchable device 20 is attached and which is mounted on the mouthpiece. Such a ring may be provided with adjustment screws or equivalent adjustment means.

The implantable pump 1 further comprises a negative pressure chamber N between the dielectric elastomer tube and the casing. The pressure in the chamber N between the inner surfaces of the casing 10 and the dielectric elastomer tube is less than 1 atmosphere. In other words, a partial vacuum is formed in the chamber N. This negative pressure exerts a radial pre-strain on the dielectric elastomer tube 20. In the absence of an electric voltage applied to the dielectric elastomer tube 20, the latter tends to expand radially as it is inflated by the pressure of the fluid in the volume 22 inside the tube. This expanded state constitutes a first stable state of the dielectric elastomer tube 20.

The amount of radial pre-strain can be adjusted to suit the application and the individual by adjusting the negative pressure within the chamber N. For example, the negative internal pressure within the casing can be defined to be less than -250mmHg, preferably less than - 300mmHg, for example in a range between -250 and -600 mmHg, preferably between -300 and -600mmHg. The exact value can be adjusted as required during or after assembly, and adapted to each tube or application.

The vacuum pump does not need to be implanted; the casing 10 is airtight so that the negative pressure created during assembly is maintained.

The flank of the casing 10 can be provided with a hole 11 for creating the partial vacuum and adjusting the internal pressure in the chamber N. The hole 11 can be provided with a seal. The hole 11 can be provided with a thread to receive a screw or any suitable closing element. A sealing means can also be considered. Alternatively, the hole 11 can be in the form of a valve with a non-return flap or check valves so that the casing can be inflated or deflated.

According to one embodiment, the pressure in the internal volume 12 of the casing can be adjusted after installation of the pump 1 on an artery. For this purpose, the hole 11 can be used to pump some of the air present in the internal volume 12 in order to adjust the radial pre-strain depending on the pressure present in the fluid in the tube, for example the patient's blood pressure.

The amount of longitudinal pre-strain depends on the elongation of the dielectric elastomer tube 20. It can therefore be independently matched to the amount of radial pre-strain determined by the pressure within the chamber 20.

The ability to independently adjust the radial and longitudinal pre-strain provides an additional degree of freedom to control both the stable state(s) of the tube and the deformations caused when a voltage is applied.

These radial and longitudinal pre-strains, created by the partial vacuum within chamber N and by the adjustable elongation of the dielectric elastomer tube 20, increase the maximum head pressure and flow rate that is achieved with a given dielectric elastomer tube 20 and at a given voltage applied to the electrodes of the dielectric elastomer tube. It also increases the range of deformation of the dielectric elastomer tube 20, thereby adapting to variations in fluid pressure within the tube. It further helps the dielectric elastomer tube reach a radial deformation close to the theoretical maximum, independently of the tube thickness, and thus to reach a higher head pressure and flow rate.

The voltage applied to the dielectric elastomer tube is preferably higher than 4kV, for better performance higher than 5kV.

To function as a pump, the device may include an artificial valve before the device. An additional artificial valve may be omitted if the device can generate the necessary pressure changes to open and close the aortic valve or another body valve.

To be implantable in a human body, the length of the pump is preferably less than 80mm, preferably less than 60mm, for example 50mm. Its diameter is preferably less than 60mm, preferably less than 40mm, for example 30mm.

Tests and prototype have shown that a pump with those dimensions can achieve a maximal flow rate of more than 2000mL/min, or even more than 4000mL/min, such as 7000mL/min or more. It can also achieve a maximal head pressure of more than 30mmHg, preferably more than 40mmHg, such as 84mmHg or more. Such values have been achieved with a dielectric elastomer tube having a thickness between 2 and 5mm, preferably between 3 and 4 mm, such as 3.6mm. Such values are compatible with applications for aortic cardiac assistance that were not achieved with prior art dielectric elastomer pumps of similar dimensions.

In order to maintain such a compact dimension, the pump preferably comprises one single dielectric elastomer tube encased by the airtight casing 10.

The present description also relates to a method of assembling the pump 1 described herein. The method comprises the step of arranging the dielectric elastomer tube 20 between the two opposite mouthpieces 30a, 30b so as to surround the corresponding connection means 31a, 31b. It further comprises the step of adjusting the mechanical strain of the stretchable dielectric elastomer tube 20 to a suitable elongation. The elongation is determined according to the case of use and may be adjusted individually for each tube.

In a preferred embodiment, the longitudinal pre-strain of the tube 20 results in an elongation of more than 25%, preferably higher than 30%, for example between 40% and 70%,

The method further comprises the steps of simultaneously clamping the stretchable device 20 and the corresponding mouthpieces 30a, 30b against the casing 10. Simultaneously here means by a single action, during a single step. The method further comprises the step of adjusting the internal pressure of the internal volume of the casing 10 so as to provide a suitable back pressure.

In the present description, the term "artery" designates any conduit, preferably a biological conduit, through which a pulsed flow of fluid, such as a biological fluid, is sent.

The flow F designates any pulsed fluid flow, i.e. a nonhomogeneous flow comprising pressure waves. The flow typically designates the blood flow resulting from the pulsations of the heart. It may also denote lymphatic flow.

According to some embodiments, the pump 1 can be provided with sensors such as pressure sensors, electrical sensors, power consumption sensors. For example, a pressure sensor may be provided in the internal casing volume 12 to determine the actual back pressure and/or to detect a potential pressure leak. The present pump 1 may comprise or be connected to suitable communication means so that the data collected by the above-mentioned sensors can be communicated outside the patient's body. Such communication means may be wired or wireless. The present pump 1 may comprise or be connected to a command unit at least adapted to control the application of voltage, including the voltage values, the duration of the application of voltage, the frequencies of the application of voltage. Such a control unit may also be adapted to detect the heart pulsation in order to synchronise the activation of the pump 1.

Although the first 13 and second 53 surfaces are represented as straight planar surfaces, this does not exclude other suitable shapes, such as concave or convex profiles, provided that the corresponding opposite surfaces 310, 311 of the mouthpieces have complementary profiles so as to hermetically clamp the mouthpieces and the stretchable device 20.

One or more of the elements described above, such as the mouthpieces 30a, 30b, the casing 10 and the lateral edges 100, may comprise a coating or surface treatment adapted to the surgical applications. The above-described cardiac assist device 1 and all its parts are adapted to conventional sterilisation processes, including temperatures above 90°C or 120°C, UV radiation, solvents.

### Reference symbols in the figures

- 1: Pump
- 10: Casing
- 11: Hole
- 12: Internal casing volume
- 13a, 13b: First surface
- 20: Dielectric elastomer tube
- 20a: Central portion of dielectric elastomer tube
- 20b: Ends of dielectric elastomer tube
- 22: Internal volume of dielectric elastomer tube
- 30a, 30b: Mouthpieces
- 31a, 31b: Compression rib
- 21a, 31b: Flanges of tubular connectors
- 40a, 40b: O-ring
- 50: Locking ring
- 51: Clamping means
- 52a, 52b: Opening
- 53a, 53b: Second surface on the locking ring
- 100a, 100b: Lateral edges
- 130a, 130b: Recess
- N: Negative pressure chamber

## Claims

1. A pump (1) implantable in a human or animal body comprising :
an airtight casing (10);
a dielectric elastomer tube (20) extending within said casing (10), arranged so that a volume (22) within said dielectric elastomer tube (20) is modified when a voltage is applied to said dielectric elastomer tube;
a negative pressure chamber (N) between said dielectric elastomer tube (20) and said casing (10);
**characterized in that** said casing comprises two lateral edges (100a, 100b) and **in that** said dielectric elastomer tube (A) is longitudinally pre-strained between said lateral edges.

2. A pump according to claim 1, wherein the amount of longitudinal pre-strain of said dielectric elastomer tube (20) is adjustable during assembly of the pump.

3. A pump according to one of the claims 1 or 2, each said opposite lateral edge (100) being provided with an opening (52a, 52b) defined by a first surface (13a, 13b),
said pump further comprising at least one mouthpiece (30a, 30b) extending at least partly outside the casing (10) through one of said openings,
said dielectric elastomer tube (20) being maintained by compression between said first surface (13a, 13b) and said mouthpiece (30a, 30b).

4. A pump according to claims 2 and 3, wherein the longitudinal position of said dielectric elastomer tube (20) between said first surface (13a, 13b) and said mouthpiece (30a, 30b) can be continuously adapted to adjust the pre-strain of the dielectric elastomer tube (20).

5. A pump according to one of the claims 3 or 4, further comprising at least one locking ring (50) with a central hole facing said opening (52a, 52b) and defined by a second surface (53a, 53b), said dielectric elastomer tube (20) being further maintained by compression between said second surface (53) and said mouthpiece (30a, 30b),
said pump further comprising clamping means (51) allowing to tight said locking ring (50) against one of said lateral edge (100) so as to clamp said mouthpiece (30a, 30b) and said stretchable device (20) between said first (13) and said second (53) surfaces.

6. A pump according to claim 5, said mouthpiece comprising two opposite surfaces (310, 311) angled with regard to each other, one of said opposite surfaces facing the corresponding first surface (13) and the second of said opposite surfaces facing the second surfaces (53).

7. A pump according to one of claims 5 to 6, wherein one or more of said first (13) and second (53) surfaces is provided with a recess (130), the pump further comprising at least one O-ring (40a, 40b) arranged in said recess (130).

8. A pump according to one of claims 1 to 7, wherein said casing (10) is provided with a closable hole (11) for creating a negative pressure within said chamber.

9. A pump according to one of claims 1 to 8, wherein said dielectric elastomer tube (20) comprises several layers of electrodes and several layers of electrosensitive polymers, the shape of which is modified upon application of a voltage between said electrodes.

10. A pump according to claim 9, the thickness of said tube being higher than 1mm, preferably between 2.4 and 4mm, more preferably between 3 and 4mm.

11. A pump according to one of the claims 9 or 10, said voltage being larger than 5kV.

12. A pump according to one of the claims 1 to 11, wherein the longitudinal pre-strain of the tube 20 results in an elongation of the tube of more than 25%, preferably higher than 30%, for example between 40% and 70%.

13. A pump according to one of the claims 1 to 12, wherein the negative pressure within said chamber is lower than -300mmHg.

14. A pump according to one of the claims 1 to 13, wherein the maximum head pressure that can be achieved is higher than 30mmHg, the maximum flow rate is higher than 2000mL/min, and wherein the length and diameter of the pump are both less than 60mm.

15. Method of assembling a pump (1) according to one of claims 4 to 14, comprising the steps of :
- arranging the dielectric elastomer tube (20) so that each of its extremities (20a, 20b) surround the corresponding mouthpiece (30a, 30b),
- pre-straining the dielectric elastomer tube (20) by adjusting its elongation between said lateral edges, and
- clamping the extremities (20b) of the stretchable device (20) and the corresponding mouthpieces (30a, 30b) between the lateral edges (100) of the casing (10) and the corresponding locking rings (50).

16. Method according to claim 15, further comprising the step of adjusting the pressure (P12) inside the casing (10) by decreasing the internal pressure up to a predetermined value.
